(19) Europäisches Patentamt
European Patent Office
Office européen des brevets

(11) Veröffentlichungsnummer: **0 186 629**
**B1**

(12) # EUROPÄISCHE PATENTSCHRIFT

(45) Veröffentlichungstag der Patentschrift: **06.12.89**

(51) Int. Cl.⁴: **C 07 C 65/03**, C 07 C 51/15

(21) Anmeldenummer: **85810607.3**

(22) Anmeldetag: **17.12.85**

(54) **Verfahren zur Herstellung von 3,5-Dialkyl-4-hydroxybenzoesäure.**

(30) Priorität: **24.12.84 US 685369**

(43) Veröffentlichungstag der Anmeldung: **02.07.86 Patentblatt 86/27**

(45) Bekanntmachung des Hinweises auf die Patenterteilung: **06.12.89 Patentblatt 89/49**

(84) Benannte Vertragsstaaten: **DE FR GB IT NL**

(56) Entgegenhaltungen: **GB-A-738 359**

(73) Patentinhaber: **CIBA- GEIGY AG, Klybeckstrasse 141, CH- 4002 Basel (CH)**

(72) Erfinder: **Pastor, Stephen D., 1080 Warburton Avenue Apt. 1C, Yonkers New York 10701 (US)**
Erfinder: **Spivack, John D., 1 Blue Jay Street, Spring Valley New York 10977 (US)**
Erfinder: **Odorisio, Paul, 269 13th Street, Palisades Park New Jersey 07650 (US)**

**EP 0 186 629 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

3,5-Dialkyl-4-hydroxybenzoesäure und ihre verschiedenen Ester sind bekannte Stabilisatoren für organische Polymere, die gegen oxidativen Abbau empfindlich sind. Von den Patentschriften, die diese Verbindungen offenbaren, können die US-Patente 3 330 859 und 3 681 431 als repräsentativ angesehen werden. In diesen Patenten werden Veresterungen von Säuren, Säurehalogeniden oder Säureanhydriden mit geeigneten Alkoholen beschrieben.

Auch andere Herstellungsverfahren sind beschrieben worden. Zum Beispiel ist die Carboxylierung von Alkalimetall-phenolaten mit $CO_2$ zur Herstellung von p-Hydroxybenzoesäuren bekannt. [A.S. Lindsey und H. Jeskey, Chem. Rev. *57*, 583 - 620 (1957)]. Die Verwendung von dipolaren, aprotischen Lösungsmitteln bei der Carboxylierung von Alkalimetallsalzen von 2,6-Dialkylphenolen ist ebenfalls beschrieben worden [siehe zum Beispiel US-3 825 593; US-4 034 006; US-4 072 707]. Bei diesen Verfahren wurde bevorzugt N,N-Dimethylformamid als dipolares, aprotisches Lösungsmittel verwendet, obwohl es die Nachteile eines hohen Siedepunktes (Kp = 153°C) und einer für den Menschen grossen Toxizität besitzt [M. Windholz, Merck-Index, 9. Auflage, Seite 3236].

Andere Lösungsmittel für die Carboxylierung von Phenolaten sind untersucht worden, aber deren Verwendung führte zu bedeutend geringeren Ausbeuten. Zum Beispiel haben T. Sakakihara und K. Haraguchi [Bull. Chem. Soc. Jpn. *53*, 279 (1980)] gezeigt, dass die Carboxylierung von Natriumphenolat in Polyethylenglykoldimethylethern äusserst geringe Ausbeuten von Hydroxybenzoesäure liefert. In der Tat wird keine Benzoesäure gebildet, wenn Natriumphenolat in 1,2-Dimethoxyethan mit $CO_2$ umgesetzt wird.

In der US-Patentschrift 4 072 707 von Grosso wird die Verwendung von 1 bis 5 Äquivalenten Alkalimetallhydrid bezogen auf 2,6-Dialkylphenol als bevorzugte Herstellungsmethode für die entsprechenden Alkalimetallsalze der 2,6-Dialkylphenole beschrieben, ungeachtet der hohen Kosten von Alkalimetallhydriden im Vergleich zu Alkalimetallhydroxiden. Die Alkalimetallsalze werden in einem dipolaren, aprotischen Lösungsmittel mit $CO_2$ behandelt und man erhält 3,5-Dialkyl-4-hydroxybenzoesäure.

Es ist wünschenswert, ein Verfahren zu entwickeln, das in bezug auf Ausbeute, Reinheit und Wirtschaftlichkeit eine Verbesserung zeigt, aber gleichzeitig die Möglichkeit bietet, extreme Reaktionsbedingungen zu vermindern und Toxizitätsprobleme auszuschliessen.

Folglich ist es das Ziel der vorliegenden Erfindung, ein verbessertes Verfahren zur Herstellung von 3,5-Dialkyl-4-hydroxybenzoesäure zur Verfügung zu stellen.

Verschiedene andere Gegenstände und Vorteile dieser Erfindung werden in den folgenden detaillierten Ausführungen sichtbar.

Die vorliegende Erfindung beschreibt ein neues und verbessertes Verfahren zur Herstellung von 3,5-Dialkyl-4-hydroxybenzoesäure aus $CO_2$ und einem Alkalimetallsalz von 2,6-Dialkylphenol. Als Lösungsmittel wird Mono- oder Polyalkylenglykolether verwendet.

Die folgenden Vorteile sind besonders relevant für diese Erfindung:

1) Verwendung von $CO_2$ bei atmosphärischem Druck und daher Vermeidung der Schwierigkeiten, die bei Hochdruckverfahren auftreten.
2) Niedrige Temperaturen und somit Vermeidung der Probleme und Kosten, die bei Hochtemperaturverfahren auftreten.
3) Homogene Phase durch Verwendung eines Lösungsmittels und dadurch Vermeidung der Schwierigkeiten, die bei heterogenen Gas-Feststoff-Werfahren auftreten.
4) Teilweiser oder völliger Ersatz der teuren Metallhydride durch Metallhydroxide.
5) Verwendung von Polyethylenglykoldialkylethern, die einen niedrigen Siedepunkt besitzen. So erlaubt die Verwendung von Ethylenglykoldimethylether (Kp 85°C) im Vergleich zu dem früher verwendeten N,N-Dimethylformamid (Kp 153°C) eine leichtere Wiedergewinnung des Lösungsmittels.
6) Die bevorzugte Verwendung von Ethylenglykoldimethylether vermeidet auch eine gesundheitliche Gefährdung durch N,N-Dimethylformamid, dessen Toxizität bekannt ist.

Das allgemeine Reaktionsschema der vorliegenden Erfindung entspricht folgender Gleichung:

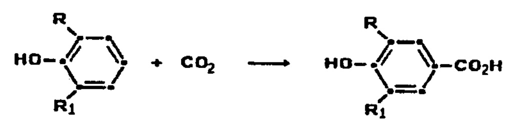 ,

worin R und $R_1$ unabhängig voneinander geradkettiges oder verzweigtes $C_1$-$C_{30}$ Alkyl sind.

$C_1$-$C_{30}$ Alkyl bedeutet beispielsweise Methyl, Ethyl, Propyl, Butyl, tert.-Butyl, Octyl, Decyl, Dodecyl, Tetradecyl, Octadecyl oder Icosyl.

Da die sterisch gehinderten Phenole bevorzugt als Stabilisatoren eingesetzt werden, ist verzweigtes $C_4$-$C_8$ Alkyl bevorzugt und besonders bevorzugt ist tert.-Butyl.

Die Reaktion erfolgt durch Umsetzung von 2,6-Dialkylphenol, welches in Gegenwart einer starken Base in das entsprechende Phenolat übergeführt wird. Als Lösungsmittel wird Mono- oder Polyalkylenglykolether verwendet, welcher auch zusammen mit einem beliebigen aromatischen Kohlenwasserstoff, wie beispielsweise Benzol, Toluol, Xylol oder ähnliches, eingesetzt werden kann. Die Mischung wird auf eine Temperatur von 30 bis 60°C erhitzt. Dann wird $CO_2$ bevorzugt unter die Oberfläche der Reaktionsmischung, eingeleitet und zwar insbesondere für eine Dauer von 0,5 bis 2 Stunden, um eine vollständige Reaktion zu gewährleisten.

Anschliessend wird zweckmäßigerweise

überschüssiges Natriumhydrid mit Alkohol zerstört, die Reaktionsmischung mit Mineralsäuren auf einen pH-Wert von 1 bis 2 eingestellt und die erhaltene 3,5-Dialkyl-4-hydroxybenzoesäure mit konventionellen Methoden aufgearbeitet.

Die bei der Reaktion eingesetzten starken Basen werden aus der Gruppe bestehend aus Ammoniumhydroxid und den Hydroxiden, Hydriden und Amiden von Alkali- und Erdalkalimetallen ausgewählt.

Die Mono- und Polyalkylenglykolether entsprechen bevorzugt der Formel

$$R_5(O\overset{R_6}{\overset{|}{C}}H(CH_2)_nCH_2)_m\text{—}OR_7,$$

worin $R_5$ und $R_7$ unabhängig voneinander $C_1$-$C_8$ Alkyl sind, $R_6$ Hydrogen oder $C_1$-$C_8$ Alkyl ist, n, 0, 1 oder 2 ist und m eine ganze Zahl zwischen 1 und 9 ist, vorzugsweise zwischen 2 und 5.

Geeignete Glykolether sind beispielsweise Ethylenglykoldimethylether, Diethylenglykoldimethylether, Diethylenglykoldiethylether, Triethylenglykoldibutylether, Tripropylenglykoldimethylether, Tetraethylenglykoldimethylether, Tetraethylenglykoldiethylether und Pentaethylenglykolmonoethylether. Die Mono- und Polyethylenglykoldialkylether sind bevorzugt Ethylenglykoldimethylether, Diethylenglykoldimethylether und Tetraethylenglykoldimethylether.

Man erhält im allgemeinen Ausbeuten von mehr als 80 %. Die erhaltenen Produkte können schon als Stabilisatoren verwendet werden und zwar sowohl in ihrer Säure- als auch ihrer Esterform.

Die folgenden Beispiele veranschaulichen die Erfindung.

**Beispiel 1:**

In einem Reaktionsgefäss befindet sich eine Suspension von 4,74 g (0,198 Mol) Natriumhydrid in 150 ml trockenem Ethylenglykoldimethylether. Es wird bei 20 bis 25°C tropfenweise eine Lösung von 20,6 g (0,1 Mol) 2,6-Di-tert.butylphenol, gelöst in 150 ml Ethylenglykoldimethylether, zugegeben. Anschliessend wird das Reaktionsgemisch 1,5 Stunden auf 50 bis 60°C erwärmt. Dann wird 20 Stunden $CO_2$ unter die Oberfläche des Reaktionsgemisches eingeleitet. Das Reaktionsgemisch wird auf 5°C abgekühlt und das überschüssige Natriumhydrid wird sorgfältig mit 30 ml Methanol zerstört. Nachdem die Wasserstoffentwicklung beendet ist, wird die Reaktionsmischung mit 1 N Salzsäure auf einen pH von 2 eingestellt und anschliessend mit 1,6 Liter Wasser verdünnt. Der erhaltene Niederschlag wird abfiltriert und *im Vakuum* getrocknet,

Das Rohprodukt wird mit 150 ml Petrolether unter Rückfluss behandelt. Man erhält 22,6 g (= 90,4 % der Theorie) eines weissen Feststoffs, der einen Schmelzpunkt von 205 - 209°C besitzt.

**Beispiel 2:**

Das Verfahren ist analog zu Beispiel 1. Es werden 4,74 g (0,198 Mol) Natriumhydrid, 20,6 g (0,10 Mol) 2,6-Di-tert.-butylphenol und 300 ml Tetraethylenglykoldimethylether eingesetzt. Man erhält 21,0 g (= 84 % der Theorie) eines weissen Feststoffs, der einen Schmelzpunkt von 205 - 208°C besitzt.

**Beispiel 3:**

In ein Reaktionsgefäss wird eine gut gerührte Mischung von 20,6 g (0,1 Mol) 2,6-Di-tert.-butylphenol, 4,0 g (0,1 Mol) Natriumhydroxid, gelöst in 3,9 g Wasser, und 250 ml Toluol gegeben. Die Reaktionsmischung wird unter Rückfluss in einer Stickstoffatmosphäre erhitzt. Das entstehende Wasser wird in einer Dean-Stark-Falle gesammelt. Nachdem 4,7 ml Wasser abgeschieden worden sind, wird die Mischung abgekühlt und 300 ml Tetraethylenglykoldimethylether werden hinzugegeben. Die Mischung wird *im Vakuum* erhitzt und 220 ml Toluol werden abdestilliert. Zu der abgekühlten Reaktionsmischung werden 0,24 g (0,01 Mol) Natriumhydrid gegeben. Die Mischung wird auf 60°C erhitzt und dann wird 20 Stunden $CO_2$ unter die Oberfläche der Reaktionsmischung eingeleitet. Die Reaktionsmischung wird auf 5°C abgekühlt und das restliche Natriumhydrid wird mit 5 ml Methanol zerstört. Nachdem die Wasserstoffentwicklung beendet ist, wird die Reaktionsmischung mit 1 N Salzsäure auf einen pH-Wert von 2 eingestellt und dann mit 1 Liter Wasser verdünnt. Anschliessend wird die Mischung zunächst zweimal mit 100 ml Ether und dann einmal mit 100 ml Chloroform extrahiert.

Die organischen Extrakte werden vereinigt und über $Na_2SO_4$ getrocknet. Das Lösungsmittel wird *im Vakuum* entfernt und der Rückstand wird mit 150 ml Petrolether unter Rückfluss behandelt. Man erhält 17,3 g (= 69 % der Theorie) eines weissen Feststoffs, der einen Schmelzpunkt von 205 - 209°C besitzt.

**Beispiel 4:**

Das Verfahren ist analog zu Beispiel 3. Es werden 20,6 g (0,1 Mol) 2,6-Di-tert.-butylphenol, 6,2 g (0,11 Mol) Kaliumhydroxid, gelöst in 7,3 g Wasser, 250 ml Toluol und 250 ml Tetraethylenglykoldimethylether eingesetzt. Nach Behandlung mit 125 ml Petrolether unter Rückfluss erhält man 10,8 g (= 43 der Theorie) eines weissen Feststoffs, der einen Schmelzpunkt von 206 - 208°C besitzt.

**Patentansprüche**

1. Ein Verfahren zum Herstellen von 3,5-Dialkyl-4-hydroxybenzoesäure, dadurch gekennzeichnet, dass 2,6-Di($C_1$-$C_{30}$alkyl)phenol und $CO_2$ in Ge-

genwart von Mono- oder Polyalkylenglykolether und in Gegenwart von Ammoniumhydroxid oder dem Hydroxid, Hydrid oder Amid eines Alkali- oder Erdalkalimetalls bei einer Temperatur von 30 bis 60°C reagieren, und dass die so erhaltene Benzoesäure isoliert wird.

2. Ein Verfahren gemäss Anspruch 1, dadurch gekennzeichnet, dass die Alkylgruppen verzweigt sind und unabhängig voneinander 4 bis 8 Kohlenstoffatome besitzen.

3. Ein Verfahren gemäss Anspruch 2, dadurch gekennzeichnet, dass die Alkylgruppen tert.-Butyl sind.

4. Ein Verfahren gemäss Anspruch 1, dadurch gekennzeichnet, dass Mono- und Polyalkylenglykolether der Formel

$$R_5(O\overset{\displaystyle R_6\atop|}{C}H(CH_2)_nCH_2)_m\text{—}OR_7$$

eingesetzt werden, worin $R_5$ und $R_7$ unabhängig voneinander $C_1$-$C_8$ Alkyl sind, $R_6$ Wasserstoff oder $C_1$-$C_8$ Alkyl ist, n 0, 1 oder 2 ist und m eine ganze Zahl zwischen 1 und 9 ist.

5. Ein Verfahren gemäss Anspruch 1, dadurch gekennzeichnet, dass der Glykolether Polyethylenglykoldialkylether ist.

6. Ein Verfahren gemäss Anspruch 1, dadurch gekennzeichnet, dass der Glykolether Ethylenglykoldimethylether, Diethylenglykoldimethylether oder Tetraethylenglykoldimethylether ist.

7. Ein Verfahren gemäss Anspruch 1, dadurch gekennzeichnet, dass die Reaktionsmischung vor der Isolierung der Benzoesäure auf einen pH-Wert zwischen 1 und 2 eingestellt wird.

**Claims**

1. A process for preparing 3,5-dialkyl-4-hydroxybenzoic acid, which comprises reacting a 2,6-di($C_1$-$C_{30}$alkyl)phenol and $CO_2$ in the presence of a mono- or polyalkylene glycol ether and in the presence of ammonium hydroxide or the hydroxide, hydride or amide of an alkali metal or alkaline earth metal at a temperature of 30 to 60°C, and isolating the benzoic acid thus obtained.

2. A process according to claim 1, wherein the alkyl groups are branched and independently of one another have 4 to 8 carbon atoms.

3. A process according to claim 2, wherein the alkyl groups are tert-butyl.

4. A process according to claim 1, wherein the mono- and polyalkylene glycol ethers employed have the formula

$$R_5(O\overset{\displaystyle R_6\atop|}{C}H(CH_2)_nCH_2)_m\text{—}OR_7$$

in which $R_5$ and $R_7$ independently of one another are $C_1$-$C_8$alkyl, $R_6$ is hydrogen or $C_1$-$C_8$alkyl, n is 0, 1 or 2 and m is an integer between 1 and 9.

5. A process according to claim 1, wherein the glycol ether is a polyethylene glycol dialkyl ether.

6. A process according to claim 1, wherein the glycol ether is ethylene glycol dimethyl ether, diethylene glycol dimethyl ether or tetraethylene glycol dimethyl ether.

7. A process according to claim 1, wherein the reaction mixture is adjusted to a pH value between 1 and 2 prior to isolation of the benzoic acid.

**Revendications**

1. Procédé de préparation d'un acide dialkyl-3,5 hydroxy-4 benzoïque, procédé caractérisé en ce qu'on fait réagir un dialkyl-2,6 phénol dont chacun des alkyles contient de 1 à 30 atomes de carbone et $CO_2$, à une température de 30 à 60°C, en présence d'un éther de mono- ou poly-alkylène-glycol et en présence d'hydroxyde d'ammonium ou de l'hydroxyde, de l'hydrure ou de l'amidure d'un métal alcalin ou d'un métal alcalino-terreux, et on isole l'acide benzoïque ainsi obtenu.

2. Procédé selon la revendication 1 caractérisé en ce que les radicaux alkyles sont ramifiés et contiennent chacun, indépendamment l'un de l'autre, de 4 à 8 atomes de carbone.

3. Procédé selon la revendication 2 caractérisé en ce que les radicaux alkyles sont chacun un radical tert-butyle.

4. Procédé selon la revendication 1 caractérisé en ce qu'on utilise un éther de mono- ou poly-alkylène-glycol répondant à la formule suivante:

$$R_5(O\overset{\displaystyle R_6\atop|}{C}H(CH_2)_nCH_2)_m\text{—}OR_7$$

dans laquelle $R_5$ et $R_7$ représentent chacun, indépendamment l'un de l'autre, un alkyle en $C_1$-$C_8$, $R_6$ représente l'hydrogène ou un alkyle en $C_1$-$C_8$, n est égal à 0, à 1 ou à 2 et désigne un nombre entier de 1 à 9.

5. Procédé selon la revendication 1 caractérisé en ce que l'éther de glycol est un éther dialkylique d'un poly-éthylène-glycol.

6. Procédé selon la revendication 1 caractérisé en ce que l'éther de glycol est l'éther diméthylique de l'éthylène-glycol, l'éther diméthylique du

diéthylène-glycol ou l'éther diméthylique du tétraéthylène-glycol.

7. Procédé selon la revendication 1 caractérisé en ce qu'on ajuste le pH du mélange réactionnel entre 1 et 2 avant d'isoler l'acide benzoïque.